# EUROPEAN PATENT SPECIFICATION

(11) **EP 3 261 592 B1**
(45) Date of publication and mention of the grant of the patent: **06.01.2021**
(21) Application number: 16754788.4
(22) Date of filing: 26.01.2016
(51) Int. Cl.: A61F 7/03, A61F 7/08, A61F 7/10, A61F 7/02

(54) **INSTANT OR DISPOSABLE COLD OR HOT PACK SYSTEM**
INSTANT- ODER EINWEG-KÜHL- ODER WÄRMEKOMPRESSENSYSTEM
SYSTÈME D'ENVELOPPEMENT CHAUD OU FROID INSTANTANÉ OU JETABLE

(30) Priority: 24.02.2015 DK 201500110
(43) Date of publication of application: 03.01.2018
(73) Proprietor: Polargate IVS, 2200 Copenhagen N (DK)
(72) Inventor: ELSUBAIHI, Rashad, 2200 Copenhagen N (DK); SAAK, Yousef Storm, 2200 Copenhagen N (DK)
(74) Representative: Herrero & Asociados, S.L.
(86) International application number: PCT/DK2016/000004
(87) International publication number: WO 2016/134720

(56) References cited:
- EP-A2- 0 068 612
- GB-A- 394 790
- US-A1- 2002 193 857
- US-A1- 2006 010 902
- US-A1- 2010 298 915
- US-A1- 2011 301 675
- US-B1- 6 666 836

## Description

### FIELD OF THE INVENTION

The present invention relates to instant and/or disposable cold packs or hot packs for sports injuries or medical care.

### BACKGROUND OF THE INVENTION

Instant or disposable hot and cold packs are often used by athletes to conveniently treat injury or used for medical care. The way they work is by taking advantage of chemicals that either absorb a lot of heat or release a lot of heat when dissolved in water. When a chemical process absorbs a lot of heat it is called endothermic; when a chemical process releases a lot of heat it is called exothermic.

Chemical cold packs contain separate components, which mix in an endothermic reaction resulting in up to 15 or 20 minutes of cold therapy. External compression of an injured area helps limit and reduce bleeding and edema and controls initial swelling by reducing the blood supply and interstitial fluids to the injured area.

In instant or disposable hot packs, calcium chloride or magnesium sulfate frequently are used because these chemicals dissolve in water exothermically. In other words, they release a lot of heat when they are dissolved in water. Hot packs can reach temperatures around 90 degrees Celsius. Hot and cold packs generally last about 20 minutes.

Patent application US2010/0298915 A1 relates to a thermotherapeutic pad having an envelope containing a thermally active material attached to a flexible flap which can be wrapped a body part and fastened in position.

On the other hand, US patent US6666836 B1 describes a device for attaching thermal dressings, such that the device is attached to the skin with an adhesive that is sufficiently strong to maintain the device in position against repeated motion.

The present invention provides an improved cold pack or hot pack system combining the two most important aspects of cold or heat therapy, temperature and compression.

### SUMMARY OF THE INVENTION

The invention is defined by the appended independent claim. Preferred embodiments of the invention are illustrated in the dependent claims.

### BRIEF DESCRIPTION OF THE DRAWINGS

Fig. 1 is a front side view of a cold pack or hot pack system according to an embodiment of the invention, and
Fig. 2 is a back side view of the cold pack or hot pack system of Fig. 1.

### DETAILED DEDCRIPTION OF THE INVENTION

Instant and/or disposable cold packs or ice packs, which are widely used for treating sports injuries, use an endothermic reaction to cool down quickly. These types of ice packs can be stored at room temperature rather than needing to be physically cooled before use. When one breaks a tube inside the pack, which may be made of a plastic material, two chemicals mix or react and absorb enough energy to produce a cooling effect. Common types may include solid ammonium nitrate dissolving in water, but instant cold packs have recently been developed without ammonium nitrate.

Disposable chemical hot packs employ a one-time exothermic chemical. The reagents are kept in separate compartments within the pack, which pack may be made of a plastic material. When the user squeezes the pack, they break and the reagents mix, producing heat. Calcium chloride or magnesium sulphate may be used, along with the water. They release heat when the chambers are broken and the chemical dissolves in the water. A hot pack can reach a temperature of 90C.

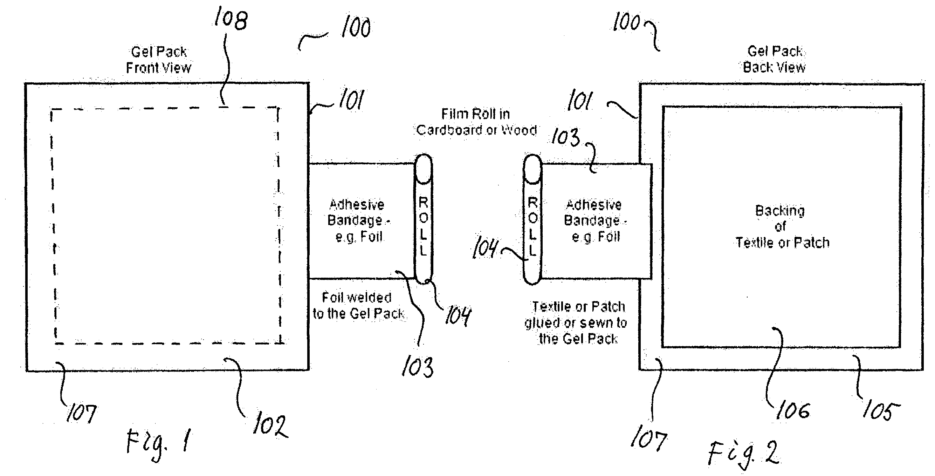

Fig. 1 is a front side view of a cold pack or hot pack system 100 according to an embodiment of the invention, and Fig. 2 is a back side view of the cold/hot pack system of 100. Figs. 1 and 2 show an instant cold pack or hot pack 101 being selected from the types discussed above, which cold/hot pack 101 is connected to an adhesive or self-adherent bandage or foil 103, where the bandage or foil 103 is partly coiled around a coiling part or roll 104. The cold/hot pack 101 has a front side wall 102 and a back side wall 105 which are sealed together. It is preferred that the front side wall 102 and the back side wall 105 are sealed and connected and along four edges. The side walls 102, 105 are preferably made of a plastic material, and the side walls 102, 105 may be sealed together by welding or by glue. A protective cover 106 is attached to the back side wall 105.

The protective cover 106 ensures that the user receives a soothing coolness, instead of a biting or bristling coldness when used for cold packs. When used for hot packs, the protective cover 106 ensures that the user is not being burnt. The protective cover 106 may cover a major part of the back side wall 105, such as at least 50%, such as at least 60%, such as at least 70%, such as at least 80%, or such as at least 90% of the surface of back side wall 105. The protective cover 106 may also cover the whole surface of the back side wall 105. However, it is preferred that a rim surface 107 or at least a part of the rim surface 107 is left uncovered by the cover 106. The sealing of the front side wall 102 and the back side wall 105 may be performed along the dotted lines 108 shown in Fig. 1 and leaving the rim surface 107. The protective cover 106 may be attached to the back side wall 105 by use of an adhesive, by welding or it may be sewed to the back side wall 105. It is preferred that the protective cover 106 is glued to the back side wall 105.

According to one embodiment of the invention, the protective cover 106 comprises a fabric or textile, such as cotton or wool. However, the protective cover 106 may also comprise other materials such as a plastic material.

According to the present invention the protective cover 106 comprises a patch or plaster. The patch or plaster is water-repellent. The patch or plaster may also or alternatively be of the type used for burns.

The bandage or foil 103 may be attached to the cold/hot pack 101 along a part of a sealed edge. It is preferred that the bandage or foil 103 is welded to a part of the rim surface 107 or to a part of the sealed edge. However, it is also within the present invention that the bandage or foil 103 is sewed or glued to a part of the rim surface 107 or to a part of the sealed edge. Fig. 2 shows an embodiment, in which the bandage or foil 103 is connected to the back side wall 105, preferably at the centre of one of the four side edges, and to a part of the rim 107, which is not covered by the protective cover 106.

According to one embodiment of the invention, a bandage 103 being a self-adherent elastic bandage is attached to the cold pack or hot pack 101. In another embodiment of the invention, a foil 103 being is attached to the cold pack or hot pack 101. This foil 103 may be a self-adherent plastic foil 103.

As illustrated in Figs. 1 and 2, a first end part of the bandage or foil 103 is attached to the cold pack or hot pack 101, and a major part of the bandage or foil 103 is rolled or coiled around a coiling part 104. The coiling part 104 may be made of plastic, cardboard, or wood. According to the invention, which is not illustrated in Figs. 1 and 2, the iength of the coiling part 104 is arger than the width of the coiled up bandage or foil 103, and the coiling part 104 may have a handle part extending below the coiled up bandage or foil 103.

## Claims

1. Therapeutic cold pack or hot pack system (100) comprising:
- an instant and/or disposable cold pack or hot pack (101) having a front side wall (102) and a back side wall (105) being sealed together along a sealed edge or edges;
- wherein the sealed edge or edges of the cold pack or hot pack (101) extend along a rim surface (107);
- an adhesive or self-adherent elastic bandage or foil (103) attached to the cold pack or hot pack (101);
wherein a first end part of the bandage or foil (103) is attached to the cold pack or hot pack (101), and wherein a major part of the bandage or foil (103) is rolled or coiled around a coiling part (104);
**characterized in that** the cold/hot pack system (100) further comprises a protective cover (106) attached to the back side wall (105);
wherein the protective cover (106) comprises a patch or plaster, which is water-repellent;
and wherein the coiling part (104) is larger than the width of the coiled up bandage or foil (103).

2. System (100) according to claim 1, wherein the protective cover (106) covers at least 50%, such as at least 60%, such as at least 70%, such as at least 80%, or such as at least 90% of the surface of back side wall (105).

3. System (100) according to any one of the claims 1-2, wherein the protective cover (106) is attached to the back side wall (105) by use of an adhesive, by welding or is sewed to the back side wall (105).

4. System (100) according to any one of the claims 1-3, wherein the protective cover (106) comprises a fabric or textile, such as cotton or wool.

5. System (100) according to claim 1, wherein the bandage or foil (103) is attached to a part of the rim surface (107) or to a part of the sealed edge.

6. System (100) according to any of the claims 1 and 5 wherein the bandage or foil (103) is welded or glued to a part of the rim surface (107) or to a part of the sealed edge.

7. System (100) according to any of the preceding claims, wherein the cold pack or hot pack (101) has four sealed edges connecting the front and back side walls (102, 105).

8. System (100) according to claim 7, wherein the bandage or foil (103) is connected to the back side wall (105), at the centre of one of the four side edges, and to a part of the rim surface (107), which is not covered by the protective cover (106).

9. System (100) according to claim 1, wherein the sealing of the front side wall (102) and the back side wall (105) is performed along dotted lines (108) and leaving the rim surface (107).

10. System (100) according to claim 1, wherein the coiling part (104) has a handled part extending below the coiled up bandage or foil (103).

11. System (100) according to claim 1, wherein the protective cover (106) comprises a plastic material.

12. System (100) according to claim 1, wherein the coiling part (104) is made of plastic, cardboard or wood.

## Patentansprüche

1. Therapeutisches Kaltkompressen- oder Heißkompressensystem (100), umfassend:
- eine Sofort- und/oder Einweg-Kaltkompresse oder Heißkompresse (101) mit einer Vorderseitenwand (102) und Rückseitenwand (105), die entlang einer versiegelten Kante oder entlang versiegelten Kanten zusammengesiegelt sind;
- wobei sich die versiegelte Kante oder die versiegelten Kanten der Kaltkompresse oder Heißkompresse (101) sich entlang einer Randoberfläche (107) erstrecken;
- eine an der Kaltkompresse oder Heißkompresse (101) befestigte klebende oder selbstklebende, elastische Bandage oder Folie (103);
wobei ein erstes Ende der Bandage oder Folie (103) an der Kaltkompresse oder der Heißkompresse (101) angebracht ist und wobei ein Hauptteil der Bandage oder Folie (103) um einen Wickelteil (104) gerollt oder gewickelt ist;
**dadurch gekennzeichnet, dass** das Kalt-/Heißkompressen-system (100) ferner eine Schutzabdeckung (106) umfasst, die an der Rückseitenwand (105) angebracht ist;
wobei die Schutzabdeckung (106) einen Flicken oder ein Pflaster umfasst, der/das wasserabweisend ist;
und wobei der Wickelteil (104) größer ist als die Breite der aufgerollten Bandage oder Folie (103).

2. System (100) nach Anspruch 1, bei dem die Schutzabdeckung (106) mindestens 50% abdeckt, wie beispielsweise mindestens 60%, wie beispielsweise mindestens 70%, wie beispielsweise mindestens 80%, oder wie beispielsweise mindestens 90% der Oberfläche der Rückseitenwand (105).

3. System (100) nach einem der Ansprüche 1-2, bei dem die Schutzabdeckung (106) unter Verwendung eines Klebstoffs, durch Schweißen an der Rückseitenwand (105) angebracht ist oder an die Rückseitenwand (105) genäht ist.

4. System (100) nach einem der Ansprüche 1-3, bei dem die Schutzabdeckung (106) einen Stoff oder ein Gewebe wie beispielsweise Baumwolle oder Wolle umfasst.

5. System (100) nach Anspruch 1, bei dem die Bandage oder Folie (103) an einem Teil der Randoberfläche (107) oder einem Teil der versiegelten Kante angebracht ist.

6. System (100) nach einem der Ansprüche 1 und 5, bei dem die Bandage oder Folie (103) an einen Teil der Randoberfläche (107) oder an einen Teil der versiegelten Kante geschweißt oder geklebt ist.

7. System (100) nach einem der vorhergehenden Ansprüche, bei dem die Kaltkompresse oder Heißkompresse (101) vier versiegelte Kanten aufweist, die die Vorder- und Rückseitenwände (102, 105) verbinden.

8. System (100) nach Anspruch 7, bei dem die Bandage oder Folie (103) mit der Rückseitenwand (105) in der Mitte einer der vier Seitenkanten und mit einem Teil der Randoberfläche (107), die nicht von der Schutzabdeckung (106) abgedeckt ist, verbunden ist.

9. System (100) nach Anspruch 1, bei dem das Versiegeln der Vorderseitenwand (102) und der Rückseitenwand (105) entlang gepunkteter Linien (108) und Auslassen der Randoberfläche (107) durchgeführt wird.

10. System (100) nach Anspruch 1, bei dem das Wickelteil (104) einen gehandhabten Teil aufweist, der sich unter die aufgerollten Bandage oder Folie (103) erstreckt.

11. System (100) nach Anspruch 1, bei dem die Schutzabdeckung (106) Kunststoffmaterial umfasst.

12. System (100) nach Anspruch 1, bei dem das Wickelteil (104) aus Kunststoff, Pappe oder Holz hergestellt ist.

## Revendications

1. Système de poche froide ou de poche chaude thérapeutique (100) comprenant :
- une poche froide ou une poche chaude instantanée et/ou jetable (101) présentant une paroi latérale avant (102) et une paroi latérale arrière (105) scellées ensemble le long d'un bord ou de bords scellé(s) ;
- dans lequel le(s) bord(s) scellé(s) de la poche froide ou de la poche chaude (101) s'étend(ent) le long d'une surface de bordure (107) ;
- une bande ou une feuille élastique adhésive ou auto-adhésive (103) fixée à la poche froide ou à la poche chaude (101) ;
dans lequel une première partie d'extrémité de la bande ou de la feuille (103) est fixée à la poche froide ou à la poche chaude (101), et dans lequel une partie majeure de la bande ou feuille (103) est enroulée ou enveloppée autour d'une partie d'enroulement (104) ;
**caractérisé en ce que** le système de poche froide/chaude (100) comprend en outre un capot de protection (106) fixé à la paroi latérale arrière (105) ;
dans lequel le capot de protection (106) comprend un patch ou plâtre, qui est hydrofuge ;
et dans lequel la partie d'enroulement (104) est plus grande que la largeur de la bande ou de la feuille enveloppée (103).

2. Système (100) selon la revendication 1, dans lequel le capot de protection (106) couvre au moins 50 %, comme au moins 60 %, comme au moins 70 %, comme au moins 80 %, ou comme au moins 90 % de la surface de la paroi latérale arrière (105).

3. Système (100) selon l'une quelconque des revendications 1 à 2, dans lequel le capot de protection (106) est fixé à la paroi latérale arrière (105) en utilisant un adhésif, par soudage ou est cousu à la paroi latérale arrière (105).

4. Système (100) selon l'une quelconque des revendications 1 à 3, dans lequel le capot de protection (106) comprend un tissu ou textile, comme le coton ou la laine.

5. Système (100) selon la revendication 1, dans lequel la bande ou feuille (103) est fixée à une partie de la surface de rebord (107) ou à une partie du bord scellé.

6. Système (100) selon l'une quelconque des revendications 1 et 5, dans lequel la bande ou feuille (103) est soudée ou collée à une partie de la surface de rebord (107) ou à une partie du bord scellé.

7. Système (100) selon l'une quelconque des revendications précédentes, dans lequel la poche froide ou la poche chaude (101) présente quatre bords scellés reliant les parois latérales avant et arrière (102, 105).

8. Système (100) selon la revendication 7, dans lequel la bande ou feuille (103) est raccordée à la paroi latérale arrière (105), au centre de l'un des quatre bords latéraux, et à une partie de la surface de rebord (107), qui n'est pas couverte par le capot de protection (106).

9. Système (100) selon la revendication 1, dans lequel le scellement de la paroi latérale avant (102) et de la paroi latérale arrière (105) est effectué le long de lignes pointillées (108) et en quittant la surface de rebord (107).

10. Système (100) selon la revendication 1, dans lequel la partie d'enroulement (104) présente une partie à poignée s'étendant en dessous de la bande ou feuille enroulée (103).

11. Système (100) selon la revendication 1, dans lequel le capot de protection (106) comprend une matière plastique.

12. Système (100) selon la revendication 1, dans lequel la partie d'enroulement (104) est réalisée en plastique, en carton ou en bois.
